# EUROPEAN PATENT SPECIFICATION

(11) **EP 1 708 613 B1**
(45) Date of publication and mention of the grant of the patent: **14.12.2011**
(21) Application number: 05702586.8
(22) Date of filing: 05.01.2005
(51) Int. Cl.: A61B 5/0402, A61B 5/11, A61B 5/024

(54) **ADAPTIVE PHYSIOLOGICAL MONITORING SYSTEM AND METHODS OF USING THE SAME**
ADAPTIVES PHYSIOLOGISCHES ÜBERWACHUNGSSYSTEM UND ANWENDUNGSVERFAHREN DAFÜR
SYSTEME DE MONITORAGE PHYSIOLOGIQUE ADAPTATIF ET PROCEDES D'UTILISATION CORRESPONDANTS

(30) Priority: 15.01.2004 US 536763 P
(43) Date of publication of application: 11.10.2006
(73) Proprietor: Koninklijke Philips Electronics N.V., 5621 BA Eindhoven (NL)
(72) Inventor: RUSSELL, James, Knox, Cleveland, OH 44143 (US); LYSTER, James, Dean, Cleveland, OH 44143 (US)
(74) Representative: Volmer, Georg
(86) International application number: PCT/IB2005/050056
(87) International publication number: WO 2005/070289

(56) References cited:
- US-A- 5 404 877
- US-A- 5 987 352
- US-A- 6 160 478
- US-B1- 6 280 409
- US-B1- 6 527 729
- US-B1- 6 662 032

## Description

The present invention relates generally to the field of physiological monitoring of patients, and more particularly, to methods and an apparatus for a physiological monitoring system with an adaptive alert mechanism.

Doctors often need round-the-clock measurements of a body parameter over a period of time to make an accurate diagnosis of a condition. Vasovagal syncopal events and arrhythmias of the heart are particularly challenging to diagnose because of their relative infrequency, sudden onset and short duration. Holter monitors are one type of ambulatory physiological monitoring systems (PMS) that are used to measure the electrical signals of a patient's heart over a period of time to detect abnormalities in the heart beat of the patient. Typically, these systems continuously monitor electrocardiography (ECG) signals for a finite test period of about 24-96 hours. By reviewing the collected ECG data in an external monitoring device, doctors may identify patients who have heart arrhythmias and who are at risk for ventricular tachycardia or other cardiac conditions.

In patients who have already been diagnosed with an arrhythmic heart condition, and who are at risk for potentially life-threatening cardiac events, it is often desired to monitor their condition over the long term. It is therefore desirable that the monitoring system be capable of long term monitoring for arrhythmic events and adequately capture such events when they occur. Since the device will be worn by the patient for potentially long durations of time, it is also desirable for the device to be compact, lightweight, mechanically robust, and unobtrusive as the patient goes about his normal daily routine of activity and rest. Traditional Holter monitors are unsuitable for such long term monitoring because of their bulky profile and relatively high power consumption levels required to power the continual ECG detection and data storage.

Ambulatory ECG monitors typically include several electrodes that are attached to the patient and a processor that acquires and processes the electrical signals into data and stores the data for later analysis. If it is functioning properly, the monitoring system is able to detect and capture cardiac event data for later retrieval and analysis. In practice, however, it is often the case that the monitoring becomes interrupted because of battery-power loss, sensor detachment problems, or other system or user errors.

Existing PMS typically employ an audible or visual alarm to alert the user of any such system malfunctions or errors. Some of the malfunctions or errors that trigger the alarm require immediate user attention, such as a malfunction requiring shutdown and restart of the PMS, signal loss due to improper sensor connection(s), or electrical interference. Other errors that trigger the alarm are less urgent (e.g., low battery power). In these prior art systems, both urgent and non-urgent types of information are communicated to the user via the alarm, whether or not the user is receptive to being disturbed.

One such conventional system is described in U.S. Patent No. 6,248,067 to Causey, Kovelman, Purvis, and Mastrototaro, and assigned to MiniMed Inc., which patent is entitled "Analyte Sensor and Holter-Type Monitor System and Method of Using the Same,".This patent describes a Holter-type recorder device equipped with a vibrator alarm or optical indicator such as a light-emitting diode (LED) that alerts the user of a system malfunction. This vibration alarm, if it remains unanswered by the user, provides additional reminders to an audio alarm. The drawback of this system is that the audio alarm may become triggered during periods of sleep or other inopportune times when the user cannot respond promptly.

US-A-5 404 877 discloses an alarm capable of sensing impedance measurements of heart, respiratory and patient motion and, from these measurements, generating an alarm signal when the measurements indicate the occurrence of a cardiac arrhythmia.

In other systems, a user of the PMS may manually change a switch setting between audible and/or silent (e.g. tactile or visual) alarm modes to prevent undesired beeping interruptions. However, manual switching represents a suboptimal solution because the burden is on the user to constantly switch between modes as he goes about his daily routine of active and inactive periods and other instances where interruptions may not be tolerated or it is necessary to conceal the presence of the PMS.

There is a need, therefore, for an improved method and apparatus for an adaptive physiological monitor that alerts the user of events indicating a system problem or malfunction when it determines that the user is in an active waking state or otherwise receptive of receiving and responding to the alarm.

In the case of PMS systems that alarm for life-threatening arrhythmia conditions, physical activity on the part of the monitored patient has some additional implications. First, physical activity may result in signal artifact that causes the monitored physiological parameter (e.g. the ECG signal) to resemble its form when some life-threatening arrhythmias (e.g. ventricular fibrillation) are present when they are in fact absent, and may generate a false positive alarm. At the same time, physical activity above a certain level may be inconsistent with the presence of the life-threatening arrhythmia or condition, because the life-threatening condition weakens the patient or renders the patient unconscious. Thus, physical activity itself, apart from its influence on the signal quality of the monitored physiological parameter, provides significant information about the patient's

cardiac status, and in combination with the ECG signals, provides a more complete assessment of the cardiac condition of a patient at a given time.

Accordingly, there is also a need for a knowledge-based PMS that inhibits the transmission of an alarm reflecting a life-threatening physiological event when the system detects that the patient is physically active.

The present invention solves these and other problems by providing a method according to claim 18 an apparatus according to claim 1.

Preferred embodiments are defined in the dependent claims Detection of arrhythmia does not form part of the invention.
FIG 1 is a perspective view of one embodiment of the physiological monitoring system of the present invention.
FIG 2 is a pictorial view showing a physiological monitoring system (PMS) according to an embodiment of the present invention on a patient.
FIG 3 illustrates a block diagram depicting the major components of the PMS according to an exemplary embodiment of the present invention.
FIG 4 is a flow chart depicting the steps performed by the physiological monitoring system to create a feedback loop that monitors a patient's physiological parameters and alerts the patient of system errors and physiological conditions in an adaptive manner based on the detected activity level of the patient.

A more complete understanding of the method and apparatus of the present invention is available by reference to the following detailed description of the embodiments when taken in conjunction with the accompanying drawings. It is worthy to note that any reference herein to "one embodiment" or "an embodiment" means that a particular feature, structure, or characteristic described in connection with the embodiment is included in at least one embodiment of the invention. The appearances of the phrase "in one embodiment" in various places in the specification are not necessarily all referring to the same embodiment. The detailed description of the embodiments which follows is intended to illustrate but not limit the invention. The scope of the invention is defined by the appended claims.

A physiological monitoring system according to the present invention comprises an adaptive system that, in addition to monitoring a particular physical characteristic of the patient for medical purposes, communicates a variety of information to the user based on a knowledge-based approach that determines whether the user is physically active. If the system detects that the patient is asleep, resting, or otherwise in a non-active state, it defers the transmission of non-urgent information until it detects that the patient is in a normal active state. On the other hand, if the system detects that the patient is in a normal active state, it inhibits, as false alarms, transmission of any urgent information that is inconsistent with the patient being in a normal active state.

Briefly, the above-described adaptive monitoring is achieved by way of an internal microprocessor having sufficient logic and data analysis capabilities to independently perform all internal control functions, including acquisition of data from sensors, processing of that data, and providing appropriate instructions to various sensors and output devices. On-board memory (e.g., static read and write memory) performs control and analysis processes and selectively stores critical information regarding a cardiac episode.

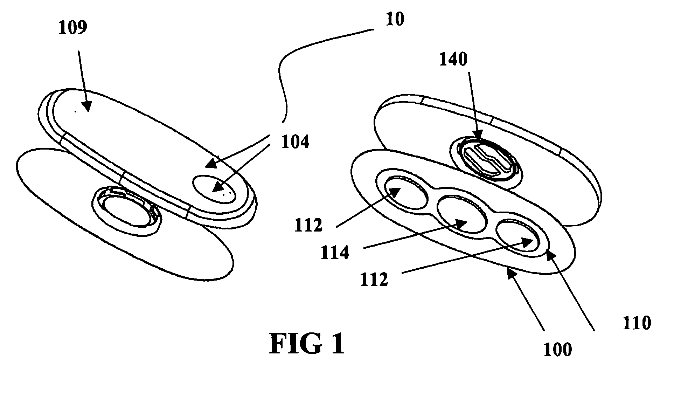

FIG 1 illustrates a perspective view of an embodiment of the monitoring system for monitoring cardiac parameters in an ambulatory setting of daily activity. The system 10 comprises a monitor 100 and sensors 110 or other transducers that are connected to the monitor 100. A wireless transmitter 109, and optionally, an event button 104 are located on the monitor 100.

The monitor 100 is designed for use with one or more sensors or transducers such as electrodes 112. As will be appreciated by one of skill in the art, a variety of sensors may be employed in the practice of this invention. With sufficient hardware and connections to the body, numerous physiologic parameters may be sensed as is pointed out in U.S. Pat. No. 5,464,434 issued to Alt and U.S. Pat. No. 5,464,431, issued to Adams et al.The physical parameters may include, but are not limited to, a patient's body movements, heartbeats, respiratory movements, snoring, and other mechanical movements and sounds detected by the sensors, respiration rate and depth (by for example, impedance plethysmography), brain waves, body temperature, and blood pressure.

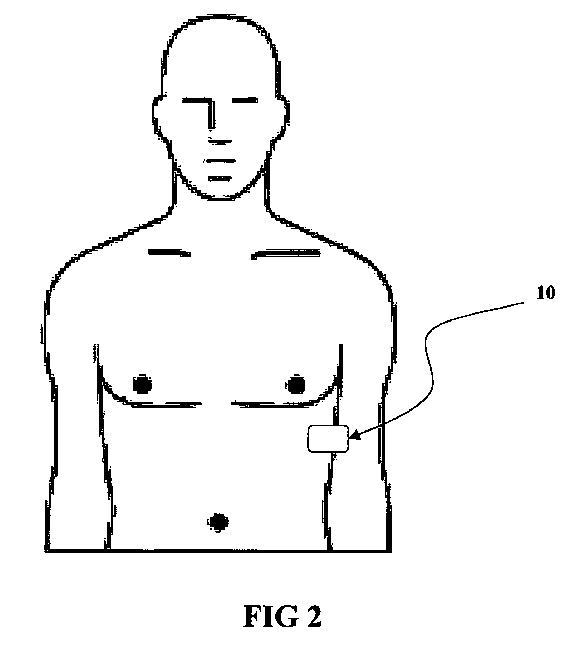

A plurality of sensors 110 may be employed simultaneously to measure the same or different physiological characteristics. For instance, typically two to six electrodes 112 may be employed to measure biological rhythmic signals such as heart rate in conjunction with an activity sensor 114 such as an accelerometer that measures the physical activity level of the patient. As shown in FIG 2, in one embodiment, at least one set of sensors 110 comprises ECG electrodes 112 that measure electrocardiograms and that are placed in contact with the patient's body so as to receive signals from the patient which are transmitted to the monitor 100. As is obvious to one of skill in the art, the electrodes may be conventional electrodes comprised of silver chloride or other compositions designed to receive analog ECG input. The system 10 may also comprise a set of activity sensors 114 that detect motion, movement, acceleration, mechanical vibrations, sound, or other indicator of physical activity on the part of the human subject.

The activity sensor 114 may comprise a piezoelectric pressure transducer, a pedometer, a vibratory or motion detector, a detector that measures residual noise generated by friction between the electrode contacts and the patient's skin, strain gage or other transducer means for measuring activity. In addition to an accelerometer, other sensors which measure physiological parameters which distinguish between resting and active states may be employed.

In one mode of the invention, the activity sensor 114 is a cantilevered suspended element which constitutes a high impedance voltage generator such as a piezoelectric element. The sensors may be incorporated into the monitor module 100 itself, or placed in close proximity to the monitor. The output of the activity sensor 114 is connected to a processor 142 contained within the monitor 100. The piezoelectric element is a passive element or sensor requiring no power to cause it to be operational. The distortion of the surface of or of the element, itself, generates the appropriate signal.

The activity sensors 114 may alternatively detect chemical or electrical changes in the patient that indicate the onset of sleep. For example, in one exemplary embodiment, the activity sensor 114 is integrated within the monitor 100 unit, as shown in FIG 2, and in other embodiments, are external to the monitor 100, such as a wrist-mounted activity sensor 114 attached to a wrist with a strap that detects electromyographic (EMG) electrical impulses produced by the wearer's wrist muscles. Such electrical impulses provide measurements of the changes in the muscular activity at the wearer's wrist, which measurements are useful in detecting drowsiness. The sensors 110 are applied to the body such that the surface of the sensors makes physical and/or electrical contact with the patient.

Although the monitor 100, and thus the sensors 110 are shown applied to the chest, one of skill in the art will appreciate that various alternative sensor construction, materials, and designs are within the scope of the present invention. The information signals from these measuring devices such as the ECG electrodes 112 and activity sensors 114 are then transmitted to the monitor 100 for amplification and processing.

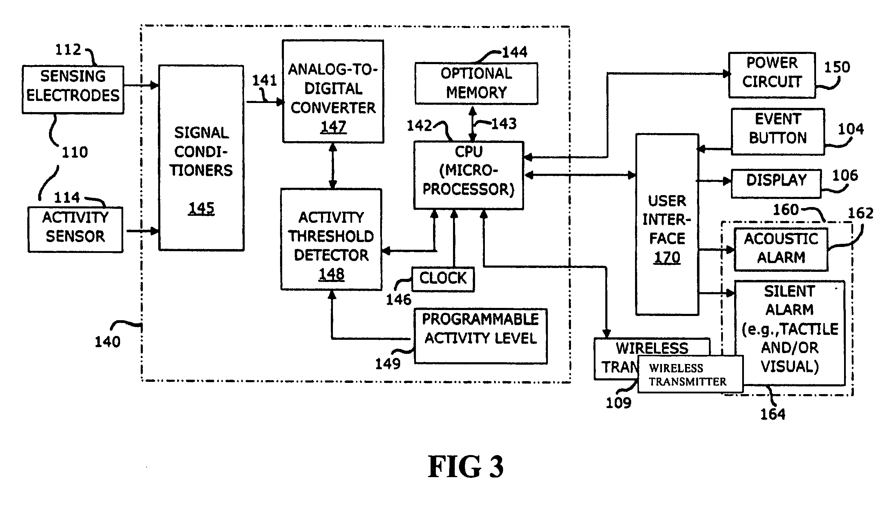

Referring now to FIG 3, which is a block diagram of the major elements of the monitoring system 10, the system includes the sensors 110 (sensor electrodes 112 and activity sensor 114), an electronics module 140, a power circuit 150, which provides power to the system 10, and may include a voltage splitter and voltage regulator in addition to a battery, an alarm 160 (acoustic alarm 162 and silent alarm such as a tactile or visual/light alarm 164), a user interface module 170, a wireless transmitter 109 or other means for communicating with an external computer, device, or medical personnel (not shown). The user interface module 170 includes an event button 104 and optionally, a user display 106 which displays status messages and system error messages to the user.

Referring now to the details of the exemplary electronics module 140 shown in block form in FIG 3, the major components of the electronic module 140 include a CPU or microprocessor 142 with an internal CPU memory 144 and an internal digital input/output circuit, signal conditioners 145, analog-to-digital converters 147, an activity threshold detector 148 input with a programmable activity level 149, and a clock 146. The microprocessor 142 performs a multitude of functions, including, but not limited to, receiving and processing signals output from the sensors 110 regarding arrhythmia conditions and checking and processing system errors. The selection and design of such circuitry and various other circuit means will be obvious to one of ordinary skill upon review of this specification.

The output of the sensors 110 such as the ECG electrodes 112 is coupled to a signal conditioning circuit 145 which filters and amplifies the output. For the purposes of this example, the present invention contemplates that the ECG electrode sensors 112 and activity sensor 114 are analog signals that are communicated to an analog-to-digital (A/D) converter and communicated to the microprocessor 40 as digital signals along signal path 141. Digital sensors may be substituted, bypassing the A/D converter 147.

Communication between the microprocessor 142 and memory/storage 144 is provided by memory line 143. The data storage (memory) device 144 is optionally included for storing the ECG signal data, pre-set threshold limits for the physiological conditions being monitored, and user input received through the user interface module 170.

In the interest of keeping the monitor 100 compact and lightweight, the memory 144 may be limited to internal CPU/microprocessor memory, which for example, may be static random access memory or "flash" memory, their equivalents, or any of the above in combination with conventional magnetic storage such as a small portable disk drive unit. If the memory comprises only an internal CPU flash memory with limited data storage capacity, the ECG signal data detected by electrodes 112 may be stored into memory in a continuous overwrite mode.

In the present embodiment, flash memory (e.g., 128K of SRAM) is provided. Under normal circumstances when no arrhythmia event is occurring, the monitoring system 10 continuously overwrites the ECG data in the CPU flash memory. When an arrhythmia event does occur, the monitoring system 10 retains the data for the short time period surrounding the event (e.g. 1 minute) in the memory for later study. As will be appreciated by one of skill, the information may be stored in reserved areas of a looping memory, preferably in identifiable memory partitions and accessed in sections or in its entirety with the appropriate external device to initiate and receive such transmissions from the monitoring system 10. Event recording using such a looping memory is detailed in the prior art such as in U.S. Patent No. 5,987,352 to Klein, Warkentin, Riff, Lee, Carney, Turi, and Varrichio, and assigned to Medtronic, Inc., which patent is entitled "Minimally Invasive Implantable Device for Monitoring Physiologic Events,"

For each physiological parameter that is being monitored by the sensors 110, an individualized baseline or threshold may be computed and stored in the RAM memory of the microprocessor 142 or an optional external memory 144. In the case of some physiological parameters, fixed threshold levels may be used. The microprocessor 142 receives status information from the memory 144 regarding these system variables. In an exemplary embodiment, information regarding the arrhythmia frequency that is pre-determined to qualify as a class 1 arrhythmia event is stored in the flash memory of the microprocessor 142. If the ECG sensors measure electrical signals that exceed the programmed arrhythmia frequency, the microprocessor 142 alerts the patient through the user interface module 170, and/or with external devices through the wireless transmitter 109. In the case of alerts reflecting states in which the patient may require assistance, or may be unresponsive, alerts may instead or additionally be directed, by means of wireless transmission to an external system, such as to an emergency responder.

The user interface 170 communicates with the microprocessor 142 of the electronics module 140 via an internal digital input/output circuit which communicates directly with the microprocessor 142 on input/output lines. For instance, when the battery power is low (e.g., depletion within 24 hours of usage), the microprocessor prompts a message on the user display 106, warning the user that the battery power needs to be recharged. The user display 106 is a visual display that may be implemented as a light-emitting diode (LED) display, a dual-colored (back to back) LED, or a conventional alphanumeric display such as a liquid crystal display (LCD). In the single LED embodiment, the microprocessor may cause the LED to flash to indicate low battery power or a system error. In a dual-colored LED embodiment, the microprocessor 142 may cause the LED to remain unlit and flash in two different colors to indicate the status of the battery power or other system functions. If an LCD is employed, the display may indicate the time of day as well as system status information.

In some embodiments, the pressing of the optional event button 104 by the ambulatory patient causes the user interface 170 to send a signal to the microprocessor 142 to trigger the acoustic alarm 162 which emits an audible alarm and optionally transmits an alarm signal to a wired or wireless transceiver or other external communication device (not shown) for contacting appropriate medical personnel. In addition, actuation of the event button 104 may cause the microprocessor 142 to record the time an event such as the suffering of chest pains or heart flutters occurred so that the ECG data recorded at that time can be flagged for closer examination. Pressing the event button 104 also causes the microprocessor 142 to store the ECG data sequence of the event in the flash memory of the microprocessor 142 for later transmission to an external monitoring device (not shown) and later study. In the event of a false alarm, the optional event button 104 may also be used to contact and to cancel the emergency help.

The acoustic alarm 162 also emits an acoustic signal when an observed cardiovascular activity or other physiological parameter surpasses a preset limit (e.g., surpassing the maximum number of arrhythmias preset by the doctor will generate an alarm). In the case of a physiological state that is inconsistent with normal physical activity on the part of the patient, if the activity monitor threshold is exceeded, such an alert may be suppressed as a false alarm.

The output of the sensors 110 (electrode sensors 112 and activity sensor 114) is coupled to a signal conditioning circuit 145, which amplifies and filters the signals, and converted to a digital format by the analog/digital converter. The signals from the activity sensor 114, once digitally processed, are input into an activity threshold detector 148, which is coupled to the A/D converter 147. The flow of signals and activity threshold detector 148 are set to a selected activity level by programmable activity level 149. The activity threshold detector 148 is connected to a control circuit in the microprocessor 142. The output of activity threshold detector 148 may be in binary form. For example, it may be a binary 1 if a threshold level of activity is sensed and a binary 0 if less than the threshold level of activity is sensed. The binary signal is fed to the control circuit of the microprocessor 142, which operates to power the alarm system 160. Multiple activity thresholds may be employed, establishing separate control levels for the separate adaptive functions of controlling non-urgent communication and urgent communication.

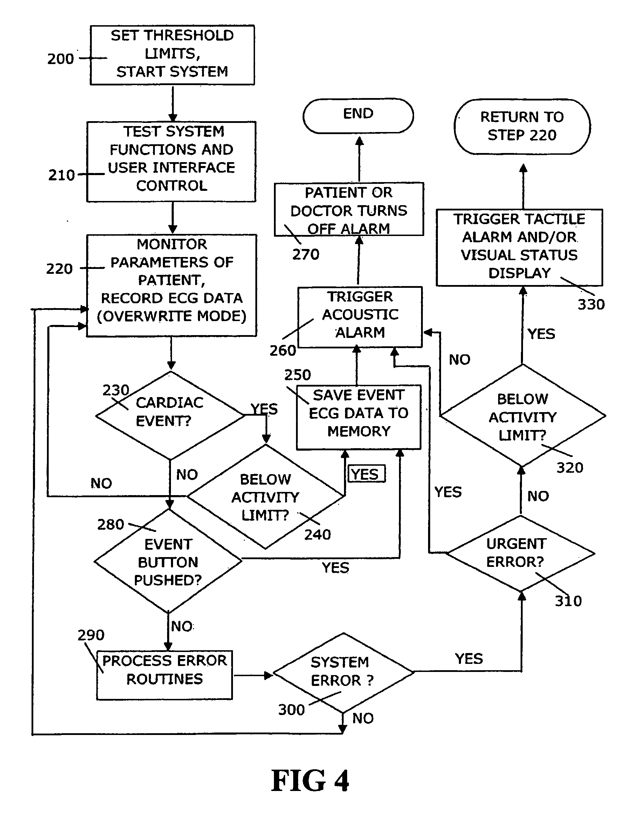

FIG 4 illustrates a flow chart of a method performed by the system 10 during adaptive monitoring of the patient according to an embodiment of the present invention. The microprocessor 142 initially sets up system variables in its memory so that the variables are proper for operation (step 200). For instance, data relating to the threshold conditions for each physiological parameter are entered into the memory of the microprocessor 142 serving the monitor 100. Thus, pre-determined baseline information regarding arrhythmia, such as the frequency and signal amplitude of electrocardiograms that would constitute a class 1 arrhythmic event are stored in the flash memory of the microprocessor 142. It is also within the scope of this invention to utilize algorithmic routines to calibrate non-static, non-preset thresholds that adapt to changing physical parameter base line information.

The system is also set so that various types of system errors, malfunctions or low power level (hereinafter collectively referred to as "errors") are categorized as either urgent or non-urgent and this information is contained in the memory. In an exemplary embodiment of the physiological monitoring system 10 of the present invention, the microprocessor 142 runs an error routine to check for errors. Based on pre-set information, it recognizes whether an error that has occurred is "urgent" (e.g., hardware or software malfunction, system crash, corrupted data, or other error that requires the user to reinitialize the system or to obtain technical servicing of the module) or "non-urgent" (e.g., signal loss from one of the several electrode sensors due to detachment from the patient's body, warning that 24 hours of battery operating time remains, or static or electrical interference issues) that will need eventual, but not immediate patient attention.

When the monitor 100 is powered up, the microprocessor 142 executes an initialization routine to prepare the monitor unit for operation (step 210). Part of the initialization routine is for the microprocessor 142 to perform power up tests of the monitor 100 and controls of the user interface 170, including the event button 104 and user display 106. If the system fails any of the power up tests, the microprocessor 142 idles and prompts status messages on the visual display through the user interface 170.

If the system checks were successfully completed, the system enables a data acquisition mode (step 220). During the data acquisition mode, the system 10 receives and processes information from the electrode sensors 112 and continuously overwrites the ECG data in the CPU flash memory 144. Although it is within the scope of this invention to do so, to conserve memory and processing power, the activity data from the activity sensors 114 is not recorded. The data acquisition mode also includes receiving and processing information about the activity level of the patient output from the activity sensor 114. As described above, the output from the activity sensor 114 is processed through an activity threshold detector.

The monitoring continues until signals from the electrode sensors exceed a pre-set threshold, and thus indicate a "cardiac event," (step 230). The microprocessor 142 determines whether the signals indicate a cardiac event of the type that is life-threatening regardless of the physical activity level of the patient, or of another type that is life-threatening only if physical activity level is below a pre-set threshold. For cardiac events that are inconsistent with normal physical activity on the part of the patient (e.g., cardiac event is of the type that should not trigger an alarm condition if it is detected while the patient is physically active), the microprocessor 142 processes the output of the activity threshold detector 148.

If the microprocessor 142 determines that the measured signal level is above a pre-programmed activity threshold for a predetermined period of time (which may be medically and experimentally determined), indicating that the patient is in an alert state, the microprocessor 142 may suppress the cardiac event alert and return the system to the monitoring state 220. However, if in addition to exceeding the pre-set threshold for the electrode sensors, the activity level is below the set variable threshold (step 240), indicating that the patient is inactive, and has probably been rendered unconscious by the cardiac event, the microprocessor 142 does not suppress the cardiac event alert.

In certain embodiments of the invention, the PMS may be programmed with additional pre-set thresholds that recognize a variety of ECG signals and patterns that indicate a cardiac event even when physical activity is detected on the part of the patient. In such instances, when ECG signals of a pre-determined type exceed the pre-set threshold, the cardiac event alerting will remain unsuppressed regardless of the patient's activity level.

When an unsuppressed cardiac event occurs, the microprocessor 142 records the time from clock 146, turns off the ECG data overwriting routine and saves ECG data relating to the cardiac event and a buffer time period before and after the event (e.g., 30 seconds before and 30 seconds after the event) in the flash memory of the microprocessor 142 for later transmission to an external monitoring device (not shown) and later study (step 250). It also signals the alarm circuit 160 to trigger the acoustic alarm 162 and sends a distress signal through the wireless transmitter 109 (step 260). The acoustic alarm continues to sound an alarm signal until it turned off by the patient or the doctor (step 270). If neither of the above events has occurred after a pre-set time interval of monitoring has occurred, the microprocessor 142 processes error routines (step 290).

At step 290, the microprocessor 142 checks for system errors and malfunctions and conducts a test for the battery power level. If any type of system error is detected during the error routine, the microprocessor 142 determines, based on the information in the memory 144, whether the error is urgent or non-urgent (step 300). If it is urgent, the microprocessor 142 sends a signal to trigger the acoustic alarm 162 and wireless transmitter 109 (step 310). However, if a non-urgent error is detected, the microprocessor 142 processes the output of the activity threshold detector 148.

If the microprocessor 142 determines that the measured signal level is above a pre-programmed activity threshold for a predetermined period of time (which may be medically and experimentally determined), indicating that the patient is in an alert state, the microprocessor 142 may sound an acoustic alarm 162 and wireless transmitter 109 to alert the patient to system malfunctions or errors requiring immediate attention. However, if the activity level drops below the set variable threshold (step 320), indicating that the patient is asleep or resting, the microprocessor 142 triggers a silent alarm such as a visual message on the user display 106 (step 330). The system 10 is designed to continue to monitor the patient normally until the activity level rises above the activity threshold, in which case, the system triggers the acoustic alarm (step 260).

Although various embodiments are specifically illustrated and described herein, it will be appreciated that modifications and variations are possible without departing from the scope of the invention. For example, specific electrodes for the monitoring system are depicted herein, yet other sensors are possible without departing from the scope of the present invention. Substitute sensors that detect physiological characteristics that function similarly to electrodes will be obvious to one of ordinary skill in the art. Moreover, while three electrodes are shown, other numbers of electrodes can be used without departing from the scope of the present invention. Furthermore, these examples should not be interpreted to limit the modifications and variations of the invention covered by the claims but are merely illustrative of possible variations.

## Claims

1. A physiological monitoring system (10) which comprises:
at least one sensor for detecting a biological signal (112), representative of a physiological characteristic of a monitor-wearing patient and generating an electrical signal representative of the biological signal;
at least one sensor for detecting the physical activity of the patient (114) and
generating an electrical signal, representative of physical activity;
processing means (142), coupled to said sensors (112, 114) for processing said electrical signals;
an activity threshold detector (148) coupled to said processing means (142) for receiving said electrical signals representative of physical activity;
means for testing system functions;
a user interface (170) for communicating information about the detected biological signal and system functions to the patient;
means for delaying or inhibiting the communication of information (109) in response to detection of an activity threshold by said activity threshold detector (148).

2. The system of claim 1, further comprising a means for programming said physical activity sensor 114 for operational control at a selected threshold of physical activity.

3. The system of claim 1, wherein the physiological characteristic sensor (112) is adapted to sense cardiac signals.

4. The system of claim 1, wherein the physiological characteristic sensor (112) comprises electrocardiography electrodes that detect biological signals representative of the heart beats of the patient.

5. The system of claim 1, wherein the physical activity sensor (114) comprises a transducer that detects chemical, electrical or mechanical characteristics of a monitor-wearing patient, representative of physical activity, including vibrations, motion, acceleration, electromyographic impulses, or sound impulses.

6. The system of claim 1, wherein the physical activity sensor (114) comprises an accelerometer, a pedometer, an electrical noise detector, electronic capacitive sensor, an electromyographic sensor, a skin impedance sensor, or a piezoelectric sensor.

7. The system of claim 1, wherein the physical activity sensor (114) is a passive transducer including a piezeoelectric element.

8. The system of claim 1, further comprising a means for wireless transmission of information (109) about the detected biological signal or system functions to a receiver external to the system.

9. The system of claim 1 adapted for recording electrocardiography signals from a patient, wherein said at least one sensor is
a plurality of sensors (110) for detecting a plurality of biological signals, each biological signal representative of a physiological characteristic of a monitor-wearing patient, wherein at least one sensor (110) comprises one or more electrocardiography electrodes (112) that sense electrocardiography signals from a patient , whereby the sensors generate an electrical signal representative of each respective biological signal;
wherein the system further comprises :
an arrhythmia threshold detector (142) coupled to the electrocardiography electrodes (112) for receiving said electrical signals representative of the electrocardiography signals and determining whether the signals are below or above a preset threshold;
wherein the activity threshold detector(148) is coupled to the activity sensor (114) for receiving said electrical signals representative of the activity level of the patient and determining whether the signals are below or above a predetermined threshold;
system error detector (142) for detecting system errors, including signal loss, electrode detachment from patient, low battery power, corrupted data, or electrical interference and determining if the detected error meets pre-determined criteria;
processor (142) for delaying or inhibiting the communication of system and biological signal information to the patient through a user interface (170) based on the detection of an activity threshold by said activity threshold detector (148), arrhythmia threshold by said arrhythmia threshold detector (142), and/or system errors by the system error detector (142).

10. The system of claim 9, wherein the user interface (170) comprises an alarm circuit (160) comprising acoustic, tactile, or visual modes of communicating information to the patient, and mode is determined by processor (142) based on whether the signals from the respective detectors (142, 148) meet pre-determined thresholds.

11. The system of claim 9, wherein processor (142) further comprises a calibration means for setting the threshold of the arrhythmia threshold detector (142) based on processing of electrocardiography signals from the patient to generate a baseline of electrocardiography information.

12. The system of claim 9, wherein the threshold of the arrhythmia threshold detector (142) is pre-programmed into a memory component (142, 144) of the system (10).

13. The system of claim 9, wherein the physical activity sensor (114) comprises a transducer that detects chemical, electrical or mechanical characteristics of a monitor-wearing patient, representative of physical activity, including vibrations, motion, acceleration, electromyographic impulses, or sound impulses.

14. The system of claim 9, wherein the physical activity sensor (114) comprises an accelerometer, a pedometer, an electrical noise detector, electronic capacitive sensor, an electromyographic sensor, a skin impedance sensor, or a piezoelectric sensor.

15. The system of claim 9, wherein the physical activity sensor (114) is a passive transducer including a piezeoelectric element.

16. The system of claim 9, wherein the arrhythmia threshold detector (142) is set at a pre-determined threshold to detect the occurrence of class 1 arrhythmia event.

17. The system of claim 9, further comprising means of wireless communication (109) to an external system, for communication of information about the patient and system state to the patient or to others.

18. A method for communicating information about a patient during ambulatory monitoring of a physiological condition of the patient comprising the steps of:
attaching a physiological monitoring system (10) to a patient;
sensing one or more selected physiological parameters of the patient;
sensing the physical activity of the patient;
comparing the sensed physical activity to a pre-set threshold to determine whether the patient is in an inactive state or in an active state;
detecting a system error to be communicated to the patient and determining whether the detected error meets pre-determined criteria;
generating an error signal based on the system error and transmitting the error signal to the patient via a user interface (170), if the patient is in the active state.

19. The method of claim 18, wherein the physiological monitoring system (10) comprises a physical activity sensor (114) comprising a transducer that detects pre-determined chemical, electrical or mechanical characteristics of a monitor-wearing patient that are representative of physical activity, wherein the characteristics comprise vibrations, motion, acceleration, electromyographic impulses, or sound impulses.

20. The method of claim 18, wherein the physiological monitoring system (10) comprises a physical activity sensor (114) comprising an accelerometer, a pedometer, a noise detector, electronic capacitive sensor, an electromyographic sensor, or a piezoelectric sensor.

21. The method of claim 18, wherein the selected physiological parameter of the patient is sensed by at least one sensor (110) comprising two or more electrocardiography electrodes (112) that sense electrocardiography signals from the patient, whereby the sensor (110) generates an electrical signal representative of the selected physiological parameter.

## Patentansprüche

1. Physiologisches Überwachungssystem (10), das Folgendes umfasst:
mindestens einen Sensor (112) zum Detektieren eines biologischen Signals, das ein physiologisches Merkmal eines Patienten, der ein Überwachungsgerät trägt, darstellt, und zum Erzeugen eines elektrischen Signals, das das biologische Signal darstellt;
mindestens einen Sensor (114) zum Detektieren der körperlichen Aktivität des Patienten und Erzeugen eines elektrischen Signals, das die körperliche Aktivität darstellt;
Verarbeitungsmittel (142), die mit den genannten Sensoren (112, 114) verbunden sind, zum Verarbeiten der genannten elektrischen Signale;
einen Detektor (148) eines Aktivitätsschwellenwertes, der mit den genannten Verarbeitungsmitteln (142) verbunden ist, zum Empfangen der genannten elektrischen Signale, die die körperliche Aktivität darstellen;
Mittel zum Prüfen von Systemfunktionen;
eine Benutzeroberfläche (170) zum Übermitteln von Informationen über das detektierte biologische Signal und die Systemfunktionen an einen Patienten;
Mittel (109) zum Verzögern oder Blockieren der Übermittlung von Informationen als Reaktion auf die Detektierung eines Aktivitätsschwellenwertes durch den genannten Detektor (148) eines Aktivitätsschwellenwertes.

2. System nach Anspruch 1, das ferner Mittel zum Programmieren des genannten Sensors (114) der körperlichen Aktivität hinsichtlich der Betriebssteuerung bei einem ausgewählten Schwellenwert der körperlichen Aktivität umfasst.

3. System nach Anspruch 1, wobei der Sensor (112) des physiologischen Merkmals so ausgelegt ist, dass er Herzsignale erkennt.

4. System nach Anspruch 1, wobei der Sensor (112) des physiologischen Merkmals EKG-Elektroden umfasst, die biologische Signale detektieren, die die Herzschläge des Patienten darstellen.

5. System nach Anspruch 1, wobei der Sensor (114) der körperlichen Aktivität einen Wandler umfasst, der chemische, elektrische oder mechanische Merkmale eines Patienten, der ein Überwachungsgerät trägt, detektiert, die die körperliche Aktivität darstellen, zu denen Vibrationen, Bewegung, Beschleunigung, elektromyografische Impulse oder Schallimpulse gehören.

6. System nach Anspruch 1, wobei der Sensor (114) der körperlichen Aktivität einen Beschleunigungsmesser, einen Schrittzähler, einen Detektor von elektrischem Rauschen, einen elektronischen kapazitiven Sensor, einen elektromyografischen Sensor, einen Hautimpedanzsensor oder einen piezoelektrischen Sensor umfasst.

7. System nach Anspruch 1, wobei der Sensor (114) der körperlichen Aktivität ein passiver Wandler mit einem piezoelektrischen Bauteil ist.

8. System nach Anspruch 1, das ferner Mittel (109) zur drahtlosen Übertragung von Informationen über das detektierte biologische Signal oder die Systemfunktionen zu einem Empfänger umfasst, der sich außerhalb des Systems befindet.

9. System nach Anspruch 1, das so ausgelegt ist, dass es EKG-Signale von einem Patienten aufzeichnen kann, wobei der genannte mindestens eine Sensor
eine Vielzahl von Sensoren (110) zum Detektieren einer Vielzahl von biologischen Signalen ist, wobei jedes biologische Signal ein physiologisches Merkmal eines Patienten, der ein Überwachungsgerät trägt, darstellt,
wobei der mindestens eine Sensor (110) eine oder mehrere EKG-Elektroden (112) umfasst, die EKG-Signale von einem Patienten erkennen,
wobei die Sensoren ein elektrisches Signal erzeugen, das jedes entsprechende biologische Signal darstellt,
wobei das System ferner Folgendes umfasst:
einen Detektor (142) eines Arrythmieschwellenwertes, der mit den EKG-Elektroden (112) verbunden ist, zum Empfangen der genannten elektrischen Signale, die die EKG-Signale darstellen, und zum Ermitteln, ob die Signale unter oder über einem voreingestellten Schwellenwert liegen,
wobei der Detektor (148) eines Aktivitätsschwellenwertes mit dem Aktivitätssensor (114) verbunden ist, zum Empfangen der genannten elektrischen Signale, die den Aktivitätsgrad des Patienten darstellen, und zum Ermitteln, ob die Signale unter oder über einem vorbestimmten Schwellenwert liegen,
einen Systemfehlerdetektor (142) zum Detektieren von Systemfehlern einschließlich Signalverlust, Ablösen von Elektroden vom Patienten, geringer Batterieleistung, fehlerhafter Daten oder elektrischer Interferenz, und zum Ermitteln, ob der detektierte Fehler vorbestimmte Kriterien erfüllt,
einen Prozessor (142) zum Verzögern oder Blockieren der Übermittlung von Informationen des Systems und der biologischen Signale an den Patienten durch eine Benutzeroberfläche (170), basierend auf der Detektierung eines Aktivitätsschwellenwertes durch den genannten Detektor (148) eines Aktivitätsschwellenwertes, eines Arrythmieschwellenwertes durch den genannten Detektor (142) eines Arrythmieschwellenwertes und/oder von Systemfehlern durch den Systemfehlerdetektor (142).

10. System nach Anspruch 9, wobei die Benutzeroberfläche (170) einen Alarmschaltkreis (160) mit akustischen, tastbaren oder visuellen Modi zum Übermitteln von Informationen an den Patienten umfasst und der Modus von dem Prozessor (142) auf der Basis davon bestimmt, ob die Signale von den entsprechenden Detektoren (142, 148) vorbestimmte Schwellenwerte einhalten.

11. System nach Anspruch 9, wobei der Prozessor (142) ferner Kalibrierungsmittel zum Einstellen des Schwellenwertes des Detektors (142) eines Arrythmieschwellenwertes umfasst, basierend auf der Verarbeitung von EKG-Signalen von dem Patienten, um eine Basis mit EKG-Informationen zu erzeugen.

12. System nach Anspruch 9, wobei der Schwellenwert des Detektors (142) eines Arrythmieschwellenwertes in einem Speicherbauteil (142, 144) des Systems (10) vorprogrammiert ist.

13. System nach Anspruch 9, wobei der Sensor (114) der körperlichen Aktivität einen Wandler umfasst, der chemische, elektrische oder mechanische Merkmale eines Patienten, der ein Überwachungsgerät trägt, detektiert, die die körperliche Aktivität darstellen, zu denen Vibrationen, Bewegung, Beschleunigung, elektromyografische Impulse oder Schallimpulse gehören.

14. System nach Anspruch 9, wobei der Sensor (114) der körperlichen Aktivität einen Beschleunigungsmesser, einen Schrittzähler, einen Detektor von elektrischem Rauschen, einen elektronischen kapazitiven Sensor, einen elektromyografischen Sensor, einen Hautimpedanzsensor oder einen piezoelektrischen Sensor umfasst.

15. System nach Anspruch 9, wobei der Sensor (114) der körperlichen Aktivität ein passiver Wandler mit einem piezoelektrischen Bauteil ist.

16. System nach Anspruch 9, wobei der Detektor (142) eines Arrythmieschwellenwertes auf einen vorbestimmten Schwellenwert eingestellt ist, um das Auftreten von Arrythmieereignissen der Klasse 1 zu detektieren.

17. System nach Anspruch 9, das ferner Mittel zur drahtlosen Kommunikation (109) mit einem externen System zum Übermitteln von Informationen über den Patienten und den Systemstatus an den Patienten und an andere umfasst.

18. Verfahren zum Übermitteln von Informationen über einen Patienten während einer ambulanten Überwachung eines physiologischen Zustands des Patienten, das die folgenden Schritte umfasst:
Anbringen eines physiologischen Überwachungssystems (10) an einem Patienten;
Erkennen von einem oder mehreren ausgewählten physiologischen Parametern des Patienten;
Erkennen der körperlichen Aktivität des Patienten;
Vergleichen der erkannten körperlichen Aktivität mit einem voreingestellten Schwellenwert um zu ermitteln, ob sich der Patient in einem inaktiven Zustand oder einem aktiven Zustand befindet;
Detektieren eines an den Patienten zu übermittelnden Systemfehlers und Ermitteln, ob der detektierte Fehler vorbestimmte Kriterien erfüllt;
Erzeugen eines Fehlersignals basierend auf dem Systemfehler und Senden des Fehlersignals an den Patienten über eine Benutzeroberfläche (170), wenn sich der Patient im aktiven Zustand befindet.

19. Verfahren nach Anspruch 18, wobei das physiologische Überwachungssystem (10) einen Sensor (114) der körperlichen Aktivität mit einem Wandler umfasst, der chemische, elektrische oder mechanische Merkmale eines Patienten, der ein Überwachungsgerät trägt, detektiert, die die körperliche Aktivität darstellen, zu denen Vibrationen, Bewegung, Beschleunigung, elektromyografische Impulse oder Schallimpulse gehören.

20. Verfahren nach Anspruch 18, wobei das physiologische Überwachungssystem (10) einen Sensor (114) der körperlichen Aktivität mit einem Beschleunigungsmesser, einem Schrittzähler, einem Rauschdetektor, einem elektronischen kapazitiven Sensor, einem elektromyografischen Sensor oder einem piezoelektrischen Sensor umfasst.

21. Verfahren nach Anspruch 18, wobei der ausgewählte physiologische Parameter des Patienten durch mindestens einen Sensor (110) mit zwei oder mehr EKG-Elektroden (112) erkannt wird, die EKG-Signale von dem Patienten erkennen, wobei der Sensor (110) ein elektrisches Signal erzeugt, das den ausgewählten physiologischen Parameter darstellt.

## Revendications

1. Système de contrôle physiologique (10) qui comprend :
au moins un capteur pour détecter un signal biologique (112), représentant une caractéristique physiologique d'un patient portant le système de contrôle et générant un signal électrique représentatif du signal biologique ;
au moins un capteur pour détecter l'activité physique du patient (114) et générant un signal électrique, représentatif de l'activité physique ;
des moyens de traitement (142) raccordés auxdits capteurs (112, 114) pour traiter lesdits signaux électriques ;
un détecteur de seuil d'activité (148) raccordé auxdits moyens de traitement (142) pour recevoir lesdits signaux électriques représentatifs d'une activité physique ;
des moyens pour tester les fonctions du système ;
une interface utilisateur (170) pour communiquer les informations sur le signal biologique détecté et les fonctions du système au patient ;
des moyens pour retarder ou inhiber la communication d'informations (109), en réponse à la détection d'un seuil d'activité par ledit détecteur de seuil d'activité (148).

2. Système selon la revendication 1, comprenant en outre des moyens pour programmer ledit capteur d'activité physique (114) pour un contrôle opérationnel, à un seuil choisi de l'activité physique.

3. Système selon la revendication 1, dans lequel le capteur de caractéristique physique (112) est adapté pour détecter les signaux cardiaques.

4. Système selon la revendication 1, dans lequel le capteur de caractéristique physique (112) comprend des électrodes d'électrocardiographie qui détectent les signaux biologiques représentatifs des battements cardiaques du patient.

5. Système selon la revendication 1, dans lequel le capteur d'activité physique (114) comprend un transducteur qui détecte les caractéristiques chimiques, électriques ou mécaniques d'un patient portant le système de contrôle, représentatif d'une activité physique, y compris des vibrations, un mouvement, une accélération, des impulsions électromyographiques, ou des impulsions sonores.

6. Système selon la revendication 1, dans lequel le capteur d'activité physique (114) comprend un accéléromètre, un pédomètre, un détecteur de bruit électrique, un capteur capacitif électronique, un capteur électromyographique, un capteur d'impédance dermique, ou un capteur piézoélectrique.

7. Système selon la revendication 1, dans lequel le capteur d'activité physique (114) est un transducteur passif comprenant un élément piézoélectrique.

8. Système selon la revendication 1, comprenant en outre un système pour une transmission sans fil d'informations (109) concernant le signal biologique ou les fonctions du système détectés à un récepteur extérieur au système.

9. Système selon la revendication 1, adapté pour enregistrer les signaux électrocardiographiques d'un patient, dans lequel ledit au moins un capteur est constitué
d'une pluralité de capteurs (110) pour détecter une pluralité de signaux biologiques, chaque signal biologique étant représentatif d'une caractéristique physiologique d'un patient portant un système de contrôle, dans lequel au moins un capteur (110) comprend une ou plusieurs électrodes d'électrocardiographie (112) qui détectent les signaux électrocardiographiques d'un patient, moyennant quoi les capteurs génèrent un signal électrique représentatif de chaque signal biologique respectif ;
dans lequel le système comprend en outre :
un détecteur de seuil d'arythmie (142) raccordé aux électrodes d'électrocardiographie (112) pour recevoir lesdits signaux électriques représentatifs des signaux électrocardiographiques et déterminer si les signaux sont en dessous ou au dessus d'un seuil prédéterminé ;
dans lequel le détecteur de seuil d'activité (148) est raccordé au capteur d'activité (114) pour recevoir lesdits signaux électriques représentatifs du niveau d'activité du patient et déterminer si les signaux sont en dessous ou au dessus d'un seuil prédéterminé ;
un détecteur d'erreur système (142) pour détecter les erreurs du système, y compris la perte de signal, le détachement de l'électrode du patient, une batterie faible, des données corrompues, ou une interférence électrique et déterminer si l'erreur détectée satisfait les critères prédéterminés ;
un processeur (142) pour retarder ou inhiber la communication du système et des informations de signal biologique au patient, par le biais d'une interface utilisateur (170), sur la base de la détection d'un seuil d'activité par ledit détecteur de seuil d'activité (148), d'un seuil d'arythmie par ledit détecteur de seuil d'arythmie (142) et/ou d'erreurs du système par le détecteur d'erreur du système (142).

10. Système selon la revendication 9, dans lequel l'interface utilisateur (170) comprend un circuit d'alarme (160) comprenant les modes acoustique, tactile ou visuel pour la communication d'informations au patient, et le mode est déterminé par le processeur (142) sur la base du fait que les signaux provenant des détecteurs respectifs (142, 148) satisfont ou non les seuils prédéterminés.

11. Système selon la revendication 9, dans lequel le processeur (142) comprend en outre un système de calibrage pour établir le seuil du détecteur de seuil d'arythmie (142) sur la base d'un traitement de signaux électrocardiographiques du patient, afin de générer une ligne de base des informations d'électrocardiographie.

12. Système selon la revendication 9, dans lequel le seuil du détecteur de seuil d'arythmie (142) est préprogrammé dans un composant de mémoire (142, 144) du système (10).

13. Système selon la revendication 9, dans lequel le capteur d'activité physique (114) comprend un transducteur, qui détecte des caractéristiques chimiques, électriques ou mécaniques d'un patient portant un système de contrôle, représentatif d'une activité physique, y compris les vibrations, un mouvement, une accélération, des impulsions électromyographiques, ou des impulsions sonores.

14. Système selon la revendication 9, dans lequel le capteur d'activité physique (114) comprend un accéléromètre, un pédomètre, un détecteur de bruit électrique, un capteur capacitif électronique, un capteur électromyographique, un capteur d'impédance dermique, ou un capteur piézoélectrique.

15. Système selon la revendication 9, dans lequel le capteur d'activité physique (114) est un transducteur passif comprenant un élément piézoélectrique.

16. Système selon la revendication 9, dans lequel le détecteur de seuil d'arythmie (142) est réglé à un seuil prédéterminé pour détecter la survenue d'un événement d'arythmie de classe 1.

17. Système selon la revendication 9, comprenant en outre des moyens de communication sans fil (109) à un système externe, pour la communication d'informations à propos du patient et de l'état du système au patient ou aux autres.

18. Procédé de communication d'informations concernant un patient pendant un contrôle ambulatoire d'une condition physiologique du patient, comprenant les étapes consistant à :
fixer un système de contrôle physiologique (10) à un patient ;
détecter un ou plusieurs paramètres physiologiques choisis du patient ;
détecter l'activité physique du patient ;
comparer l'activité physique détectée à un seuil prédéterminé pour déterminer si le patient est dans un état inactif ou dans un état actif ;
détecter une erreur de système à communiquer au patient et déterminer si l'erreur détectée satisfait des critères prédéterminés ;
générer un signal d'erreur, sur la base de l'erreur système et transmettre le signal d'erreur au patient par le biais d'une interface utilisateur (170), si le patient est dans un état actif.

19. Procédé selon la revendication 18, dans lequel le système de contrôle physiologique (10) comprend un capteur d'activité physique (114) comprenant un transducteur qui détecte des caractéristiques chimiques, électriques ou mécaniques prédéterminées d'un patient portant un système de contrôle, qui sont représentatives d'une activité physique, dans lequel les caractéristiques comprennent les vibrations, un mouvement, une accélération, des impulsions électromyographiques, ou des impulsions sonores.

20. Procédé selon la revendication 18, dans lequel le système de contrôle physiologique (10) comprend un capteur d'activité physique (114) comprenant un accéléromètre, un pédomètre, un détecteur de bruit, un capteur capacitif électronique, un capteur électromyographique, ou un capteur piézoélectrique.

21. Procédé selon la revendication 18, dans lequel le paramètre physiologique choisi du patient est détecté par au moins un capteur (110) comprenant deux électrodes d'électrocardiographie ou plus (112) qui détectent les signaux d'électrocardiographie provenant du patient, moyennant quoi le capteur (110) génère un signal électrique représentatif du paramètre physiologique choisi.
